# EUROPEAN PATENT APPLICATION

(11) **EP 1 674 092 A1**
(43) Date of publication of application: **28.06.2006**
(21) Application number: 04773264.9
(22) Date of filing: 17.09.2004
(51) Int. Cl.: A61K 31/198, A61K 31/401, A61K 31/7076, A61P 3/04, A61P 43/00, A23L 1/30, A23L 1/305

(54) **AMINO ACID COMPOSITIONS**

(30) Priority: 19.09.2003 JP 2003328600
(71) Applicant: RIKEN, Wako-shi, Saitama 351-0198 (JP); Abe, Takashi, Saitama 3350003 (JP)
(72) Inventor: ABE, Takashi, 3350003 (JP)
(74) Representative: Vossius & Partner
(86) International application number: PCT/JP2004/013620
(87) International publication number: WO 2005/027898

(57) **Abstract**

The present invention herein provides an amino acid composition which consists essentially of (1) glycine, (2) at least one member selected from the group consisting of proline and alanine, and (3) at least one member selected from the group consisting of glutamine and glutamic acid; as well as such an amino acid composition which further comprises at least one member selected from the group consisting of CoA, acetyl CoA, and pyruvic acid. This amino acid composition has an effect of accelerating the combustion of the body fat.

## Description

### Technical Field

The present invention relates to an amino acid composition obtained on the basis of the knowledge concerning the composition composed of amino acids included in the saliva secreted by the larvae of wasps (belonging to the genus Vespa) and more specifically to an amino acid composition which can accelerate the combustion of body fat.

### Background Art

The inventors of this invention have conducted various studies of the saliva secreted by the larvae of various kinds of wasps belonging to the genus Vespa and have already proved that the nutritive liquid originated from the wasps can control the formation of substances involved in the fatigues, prevent the reduction of the blood sugar level and improve the capacity of locomotion, during exercise (see Patent Document 1 specified below). The inventors of this invention have further proved that the working mechanism of the same is to promote the conversion of body fat into the energy required for exercise (see Non-Patent Document 1 specified below). It has also been suggested that the VAAM (Vespa Amino Acid Mixture) as the principal component of the nutritive liquid has a variety of effects such as the recovery from the fatigues accompanied by the physical exercise, in addition to the foregoing effects (see, Patent Document Nos. 2 to 5 specified below).

On the other hand, it has been well known that the physical exhaustion due to the physical exercise would result in the significant destruction or loss of the amino acid balance in the blood (see Non-Patent Document 2 specified below). It has been believed that this would be caused as a result of the weariness and destruction of the body tissues due to the stresses associated with the physical exercise. However, there has not yet been focused on the physiological meanings and significance thereof until now.

The inventors of this invention have further investigated the blood amino acid concentration observed after the practice of exercise and the amino acid composition of VAAM and have thus found that the amino acid composition of VAAM has an extremely high correlation with the blood amino acids whose concentrations are reduced in proportion to the fatigues due to the practice of exercise. In other words, it has been proved that the amino acids, which are reduced in considerably high rates due to the fatigue, are included in VAAM in proportional higher amounts. Accordingly, it would be recognized that the supplementation of these amino acids is indispensable to the improvement of the motor functions and the recovery from the fatigues (see Patent Document 6 specified below). On the other hand, it has likewise been proved that trehalose can markedly increase the concentration of non-esterified fatty acid (NEFA) in the mouse serum during the movement thereof (see Patent Document 7 specified below). In this respect, the nutritive liquid of the wasps contains trehalose in a substantial amount (see Non-Patent Document 3 specified below). Thus, it has been found that the simultaneous administration of trehalose and VAAM permits the more intensive improvement of the motor functions (see Patent Document 8 specified below).

As has been discussed above, VAAM may serve to promote the process for converting the body fat into the energy required for the movement or exercise under the aerobic continuous motion. This function of VAAM would be quite effective for the motions over a long period of time such as a marathon race, but it is not always effective for the strong motional loads within a quite short period of time. For this reason, there has been a need for the development of an amino acid composition which can promote the combustion of the body fat and which would be more effective for such high-load motions within a quite short period of time.
Patent Document 1: Japanese Patent No. 2,518,692;
Patent Document 2: Japanese Un-Examined Patent Publication (hereunder referred to as "JP-A") Hei 4-95026;
Patent Document 3: JP-A-Hei 4-112825;
Patent Document 4: JP-A-Hei 6-336426
Patent Document 5: JP-A-Hei 6-336432
Patent Document 6: JP-A-Hei 9-249556 (US-BA-6224861; EP-B1-873754);
Patent Document 7: JP-A-Hei 5-186353
Patent Document 8: JP-A-2000-72669 (US-BA-6287757; EP-A1-983726);
Non-Patent Document 1: ABE et al., Jap. J. Physical Fitness & Sports Med., 1995, 44:225;
Non-Patent Document 2: T. Bazzarre et al., J. Am. Collage Nutr., 1992, 11:531;
Non-Patent Document 3: ABE et al., Comp. Biochem. Physiol., 1991, 99C:79.

### Disclosure of the Invention

Accordingly, it is an object of the present invention to provide an amino acid composition having such an effect that it can promote the combustion of the body fat. In this respect, the foregoing desired effect of the present invention has not yet sufficiently been achieved by the conventional amino acid compositions such as VAAM.

Thus, the present invention herein provides an amino acid composition such as those detailed below:
1. An amino acid composition comprising (1) glycine; (2) at least one member selected from the group consisting of proline and alanine; and (3) at least one member selected from the group consisting of glutamine and glutamic acid.
2. An amino acid composition comprising glycine, proline, alanine, glutamine, and glutamic acid.
3. An amino acid composition comprising glycine, alanine, glutamine, and glutamic acid.
4. An amino acid composition comprising glycine, proline, glutamine, and glutamic acid.
5. An amino acid composition comprising glycine, proline, alanine, and glutamic acid.
6. An amino acid composition comprising glycine, proline, alanine, and glutamine.
7. An amino acid composition comprising glycine, alanine, and glutamine.
8. An amino acid composition comprising glycine, alanine, and glutamic acid.
9. An amino acid composition comprising glycine, proline, and glutamine.
10. An amino acid composition comprising glycine, proline, and glutamic acid.
11. The amino acid composition as set forth in any one of the foregoing items 1 to 10, wherein it further comprises CoA (coenzyme A).
12. The amino acid composition as set forth in any one of the foregoing items 1 to 11, wherein it further comprises acetyl CoA (acetyl-coenzyme A).
13. The amino acid composition as set forth in any one of the foregoing items 1 to 12, wherein it further comprises pyruvic acid.

The amino acid composition of the present invention permits the activation of the TCA cycle by making the following three metabolic cycles simultaneously operate so that the combustion of the body fat is initiated within a short period of time and that the combustion thereof can simultaneously be promoted from the viewpoint of the combustion time and the amount of the consumed body fat while making use of proline, glutamine, glutamic acid, alanine and glycine. The foregoing three metabolic cycles comprise those designated as the glutamine-proline, alanine and glycine cycles, respectively (these three cycles are hereunder referred to as "amino acid engine" comprehensively). For this reason, the amino acid composition would, for instance, be effective for the reduction of the fatigues accompanied by the physical exercise, the improvement of the performance of each individual and the dietary cure.

### Brief Description of the Drawings

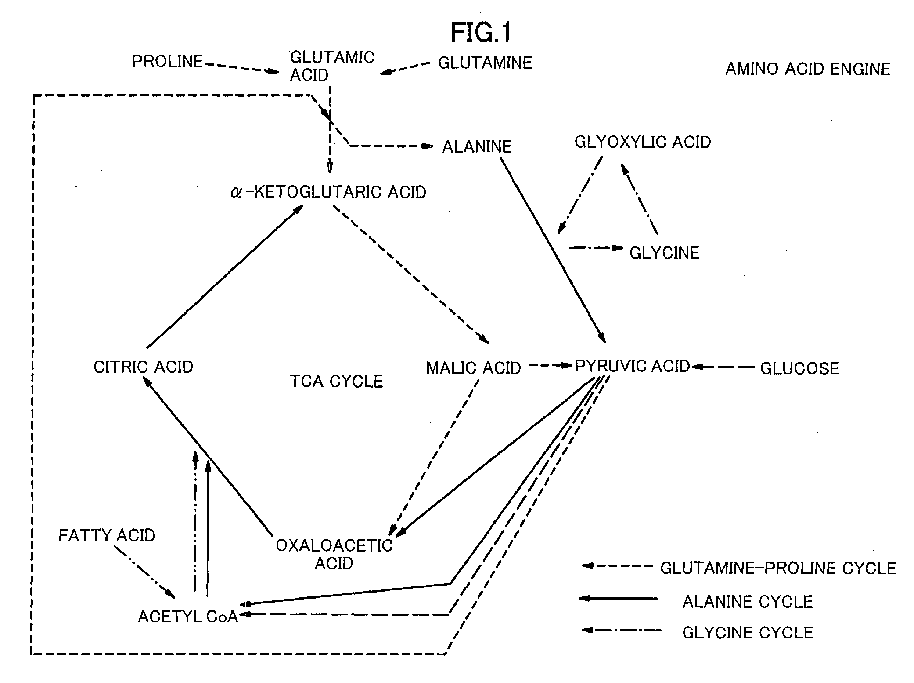

Figure 1 is a diagram for the explanation of the amino acid engine according to the present invention.
Figure is a diagram showing the results observed when orally administering, to mice, a liquid nutrient A, VAAM, proline, glutamine, alanine, and glycine and then forcing them to swim under the application of a load of 0.3 g.

### Best Mode for Carrying Out the Invention

For the combustion of the body fat, it is in general necessary to take moderate exercise over a long period of time (usually not less than 20 minutes for men) and this is because the metabolism of body fat through the combustion thereof would take place only when the TCA cycle is properly operated according to the theory of energy metabolism. The present invention has been completed on the basis of such a finding that the simultaneous operation of three cycles designated as the glutamine-proline, alanine and glycine cycles, respectively (these three cycles are hereunder referred to as "amino acid engine" comprehensively) would certainly permit the activation of the TCA cycle to thus initiate the combustion of the body fat within a short period of time and to simultaneously promote the combustion thereof from the viewpoint of the combustion time and the amount of the consumed body fat, while making use of proline, glutamine, glutamic acid, alanine and glycine. In other words, the present invention has been completed on the basis of the knowledge that proline, glutamine, glutamic acid, alanine and glycine as well as pyruvic acid, acetyl CoA and CoA are compounds having an ability to activate the TCA cycle and accordingly, the composition of the present invention is effective for the reduction of the fatigues accompanied by the physical exercise, the improvement of the performance of each individual and the dietary cure.

The combustion of body fat generates a large quantity of energy. However, it is quite difficult to combust the body fat. The body fat is the reserve energy generated as a result of the activity of life and possessing a number of advantages or, so to speak, an entity, having an ideal form, of life energy suitable for storage in order to provide necessary energy when one is on the point of dying. Therefore, the hardly combustible characteristics of the body fat should be considered to be one of the properties peculiar to the body fat. This would be resulted from the metabolic mechanism thereof.

As has been shown in Fig. 1, a series of cyclic reactions called TCA cycle (or Krebs cycle), which is composed of 10 kinds of organic acids and which constitutes the field of producing energy of life, take in one molecule of acetic acid and decompose the same into carbon dioxide and water to thus produce ATP. A sufficient quantity of oxaloacetic acid should be present in order that acetic acid (acetyl CoA) is taken in the TCA cycle. When sugar is decomposed, pyruvic acid is produced, acetyl CoA and oxaloacetic acid are synthesized starting from this product and α-keto-glutaric acid is likewise produced through the amino acid rearrangement thereof. Accordingly, several compounds taking part in the TCA cycle are also formed. For this reason, when sugar is decomposed, the TCA cycle reaction may easily be initiated.

Contrary to this, when a fatty acid is decomposed, only acetyl CoA is formed. In this case, oxaloacetic acid is not produced, which is required for the incorporation of the resulting acetyl CoA into the cycle reaction and therefore, the reaction in fact proceeds only at a quite slow rate (the reaction does not proceed at all in theory). Accordingly, the body fat always has such metabolic characteristics peculiar thereto that it cannot be combusted easily. The body fat is quite effective as storage energy, as has been discussed above, but it is quite inconvenient in such cases where a large quantity of energy should efficiently be supplied immediately under various circumstances, for instance, during taking exercise. In other words, the reactions involved in the TCA cycle must be initiated in advance to combust the body fat efficiently. To this end, it would be considered to be sufficient to produce pyruvic acid in slight excess; to produce various kinds of organic intermediates which may take part in the TCA cycle; or to partially activate the cycle reactions. Thus, conditions required for making the combustion of the body fat quite easy may completely be established.

Incidentally, the composition of VAAM is characterized in that it has high contents of proline and glycine. In case of insects having an ability to fly, proline is converted into the energy required for flying motions. In the metabolism observed for mitochondria, proline serves to supplement intermediate metabolites to the TCA cycle. Proline is converted into glutamic acid and can produce 5 molecules of ATP during this process. Moreover, the resulting glutamic acid reacts with pyruvic acid and undergoes amino acid rearrangement for the formation of alanine and α-ketoglutaric acid as a result of the rearrangement. α-Ketoglutaric acid serves as an enzyme in the TCA cycle and is converted into pyruvic acid through succinyl CoA, fumaric acid and malic acid. Thus, 8 molecules of ATP and one molecule of GTP are formed from one molecule of glutamic acid and pyruvic acid and when the proline metabolic circuit or loop is completed, 13 molecules of ATP are formed in all. The pyruvic acid finally formed again reacts with glutamic acid or it is repeatedly reused without leaving the metabolic cycle and accordingly, alanine is accumulated. The inventor of this invention recognizes that the reactions involved in the TCA cycle are partially activated by a series of metabolic reactions (glutamine-proline cycle) starting from proline or glutamine and accordingly, the combustion of the body fat is promoted.

In this respect, to operate the TCA cycle completely, the production of oxaloacetic acid should be produced subsequent to the production of pyruvic acid in excess. The glutamine-proline cycle never results in any surplus and accumulation of pyruvic acid from the stoichiometrical standpoint. Until now, as to the ATP production, the glutamine-proline cycle has been investigated in detail, but the cycle has scarcely been interested in the way how to control the overall cycle. Thus, the inventor has taken notice of the following facts that the reactions involved in the glutamine-proline cycle are irreversible ones like those involved in the TCA cycle and that this cycle undergoes the accumulation of alanine as the final product.

However, the reaction of alanine- α-ketoglutaric acid aminotransferase is a reversible one, which can produce alanine and accordingly, the production of α-ketoglutaric acid, which is conjugately produced in this reaction, is also inhibited as the accumulation of aniline proceeds. This correspondingly results in the inhibition of the whole glutamine-proline cycle and accordingly, it would be an indispensable condition for the promotion and acceleration of this glutamine-proline cycle to avoid any accumulation of alanine. Regarding the conversion of alanine, the inventor has investigated a reaction thereof for the formation of pyruvic acid and glycine through the amino group-rearrangement into glyoxylic acid, while using an alanine-glyoxylic acid amino transferase (2.6.1.44). Moreover, this reaction is irreversible one. Therefore, it would be quite suitable for not only the removal of alanine from the glutamine-proline cycle, but also the supply of pyruvic acid for operating the remaining half of the TCA cycle.

The inventor of this invention considers that cytochrome C surely takes part in the conversion of glycine into glyoxylic acid which is required for the operation of this alanine cycle. This is supported by the fact that cytochrome C is an enzyme present in the field of the TCA cycle. In addition, the alanine cycle is conjugated with this glycine cycle to thus produce fresh pyruvic acid and therefore, this would permit the continuation of the reactions involved in the TCA cycle subsequent to those for the production of oxaloacetic acid. The inventor has thus concluded that the TCA cycle is entirely operated and as a result, the combustion of the body fat is accelerated.

For this reason, the inventor thus designated the foregoing glutamine-proline, alanine and glycine cycles as "amino acid engine" comprehensively (see Fig. 1). This would play a role like a starter for the TCA cycle and it not only activates the initial metabolism, but also promotes the combustion of the body fat. This would also permit the demonstration of the functions of VAAM which has high contents of, in particular, proline and glycine.

In the amino acid composition according to the present invention, the contents of the effective components preferably range from 0 to 50 mole% and more preferably 10 to 25 mole% for proline; preferably 0 to 40 mole% and more preferably 20 to 40 mole% for glycine; preferably 0 to 40 mole% and more preferably 20 to 40 mole% for glutamine; preferably 0 to 50 mole% and more preferably 20 to 30 mole% for alanine; and preferably 0 to 25 mole% and more preferably 5 to 15 mole% for glutamic acid. In addition, the content of pyruvic acid preferably ranges from 0 to 30 mole% and more preferably 10 to 20 mole%; that of acetyl CoA preferably ranges from 0 to 10 mole% and more preferably 5 to 10 mole%; and that of CoA preferably ranges from 0 to 20 mole% and more preferably 5 to 15 mole%.

The amino acid composition of the present invention may further comprise naturally-occurring amino acids in addition to the foregoing amino acids in small amounts, for instance, not more than 5 mole%. The composition may likewise comprise taurine (Tau) (in an amount preferably not more than 3 mole%); ethanolamine phosphate (P-EtAm) (in an amount preferably not more than 2 mole%); cystine (Cys) (in an amount preferably not more than 0.5 mole%); β-alanine (β-Ala) (in an amount preferably not more than 1 mole%); γ-aminobutyric acid (GABA) (in an amount preferably not more than 0.5 mole%); ornithine (Orn) or ethanolamine (EtAm) (in an amount preferably not more than 3 mole%); ammonia (NH₃) (in an amount preferably not more than 2 mole%); 1-methylhistidine (1-MeHis) (in an amount preferably not more than 3 mole%); 3-methyl-histidine (3-MeHis) (in an amount preferably not more than 1 mole%). The amino acids used in the amino acid composition of the present invention are particularly preferably those in the L-configuration.

When preparing the amino acid composition of the present invention, it is sufficient that each of the aforementioned commercially available amino acids is admixed with the basic amino acid composition in each desired rate specified above.

In general, it is sufficient that these amino acid components in powdery states are uniformly mixed together to thus form a composition and that the resulting composition is, if necessary, dissolved in distilled water. The temperature used in the preparation and storage of the composition of the present invention is not restricted to any particular range, but the composition is preferably prepared and stored at a temperature of not higher than room temperature. The composition of the present invention tastes slightly bitter and when orally administered to mice, it does not show any toxicity even at a dose of 20 g/kg and accordingly, the LD₅₀ value thereof is much higher than 20 g/kg.

The shape of the amino acid composition of the present invention is not restricted to any particular one. The amino acid composition of the present invention is useful as, for instance, a medicine or a food such as a beverage.

When using the composition as a medicine, the dosage form thereof is not likewise limited to any particular one and the composition may be administered through any currently used routes of administration, for instance, oral administration, per rectal administration, administration through injection, and administration of a transfusion solution. When the composition is orally administered, it may be used in the form of a pharmaceutical preparation such as a tablet, a capsule, a powder, a troche or a syrup, which is prepared from the composition per se having the foregoing composition, or prepared from a mixture of the composition with a pharmaceutically acceptable carrier or excipient. In this respect, however, the composition is preferably administered through the oral route in the form of, for instance, a liquid preparation since the absorption of the composition requires a long period of time when it is administered in the form of a solid preparation such as a tablet or a powder. In this case, the composition is preferably administered in the form of an aqueous solution together with appropriate additives, for instance, a salt such as sodium chloride, a buffering agent and/or a chelating agent. Alternatively, when it is administered in the form of an injection, for instance, an appropriate buffering agent and/or an isotonicity are incorporated into the composition, the resulting mixture is then dissolved in sterilized distilled water to thus give an aqueous solution or injection and the solution is intravenously administered through, for instance, intravenous drip infusion.

When the composition is used as a food, it may be formed into a drink by the addition of an appropriate flavoring agent, such as a refreshing beverage or a powdered beverage, or it may likewise be formed into, for instance, a jelly, a candy, a chocolate, a starch jelly, and confectionery to which the composition of the present invention is added. The composition may likewise be formed into a capsule or a tablet produced using the powdered composition obtained by, for instance, the spray drying, freeze-drying or microfine powder-preparation technique.

Further, the composition of the present invention likewise preferably comprises vitamins such as those belonging to the groups of vitamins A and B.

The composition of the present invention has an extremely low toxicity and therefore, it can be administered in a quite wide dose range. The dose thereof may vary depending on the route of administration selected, each particular purpose of the use thereof, but in general ranges from 1 to 12 g/dose, 3 to 18 g/day and preferably 1 to 4 g/dose, 3 to 12 g/day, and about 3 to 8 g for the daily habitual injestion, as expressed in terms of the solid content of the composition.

The composition of the present invention is effective for the reduction of the fatigues accompanied by the physical exercise, the improvement of the performance of each individual and the dietary cure and it would be sufficient that an appropriate amount of the composition is administered properly depending on any particular purpose. When the composition is used as, for instance, a supplementary liquid to be administered before the initiation of the exercise and during and after the exercise, it is used in the form of an aqueous solution having a concentration ranging from 0.8 to 3.8% by mass and the solution is suitably administered in an amount ranging from 200 to 500 ml over one to three times a day. When using the same as an injection, it is used in the form of an aqueous solution having a concentration ranging from 0.8 to 3.8% by mass and the solution is suitably administered through injection in an amount ranging from 100 to 400 ml/dose and preferably 150 to 300 ml/dose.

### Examples

The present invention will hereunder be described in more specifically with reference to the following Test Examples and Examples, but the present invention is not restricted to these specific Examples at all.

### Examples 1 to 20

The following components listed in Table 1 were mixed together in ratios (molar ratios) likewise specified in Table 1 to thus prepare each amino acid composition according to the present invention.

**Table 1**

| Ex. No. | Gly | Pro | Ala | Gln | Glu | Acetyl CoA | CoA | Pyruvic acid |
|---|---|---|---|---|---|---|---|---|
| 1 | 25 | 20 | 25 | 25 | 5 | | | |
| 2 | 25 | | 25 | 40 | 10 | | | |
| 3 | 30 | 25 | | 40 | 5 | | | |
| 4 | 30 | 30 | 30 | | 10 | | | |
| 5 | 25 | 25 | 25 | 25 | | | | |
| 6 | 30 | | 30 | 40 | | | | |
| 7 | 40 | | 50 | | 10 | | | |
| 8 | 40 | 20 | | 40 | | | | |
| 9 | 40 | 50 | | | 10 | | | |
| 10 | 40 | | 45 | | 10 | 5 | | |
| 11 | 40 | 20 | | 35 | | | 5 | |
| 12 | 40 | 45 | | | 10 | | | 5 |

### Test Example

Animals: To ddY♂ (6-8-week-old) mice which had been fasted for 12 to 16 hours, there were orally administered a variety of amino acid-containing (2% by mass) liquid nutrients in an amount of 37.5 *µ*l/g (body weight), they were then allowed to stand for 30 minutes and they were subsequently forced to swim in a water-flowing pool maintained at 35°C. In case of the swimming under the application of a load, a weight of 0.3 g was attached to the tail of each mouse. The blood was collected from each mouse immediately after the animal was forced to swim for 30 minutes and it was then analyzed for the metabolites in blood.

Liquid Nutrients: There were used a preparation A (the preparation prepared in Example 5) having the following amino acid composition and VAAM, proline, glutamine, alanine, and glycine.

**Table 2**

| Amino acid | VAAM | Preparation A |
|---|---|---|
| Asp | 0.17 | |
| Thr | 7.03 | |
| Ser | 2.14 | |
| Glu | 3.86 | |
| Pro | 17.03 | 25.0 |
| Gly | 11.76 | 25.0 |
| Ala | 4.47 | 25.0 |
| Val | 5.63 | |
| Cys | | |
| Met | 0.7 | |
| Ile | 4.87 | |
| Leu | 6.61 | |
| Tyr | 8.86 | |
| Phe | 5.19 | |
| Lys | 10.34 | |
| Trp | 3.68 | |
| His | 3.27 | |
| Arg | 5.02 | |
| Gln | | 25.0 |

### Blood Free Fatty Acid Value:

The blood of each test animal was collected, followed by allowing it to stand for 30 minutes, subjecting the blood to centrifugation at 3000 rpm to thus obtain a supernatant (serum), which was used for the determination of the blood free fatty acid value. The determination was carried out using the following enzymatic technique:

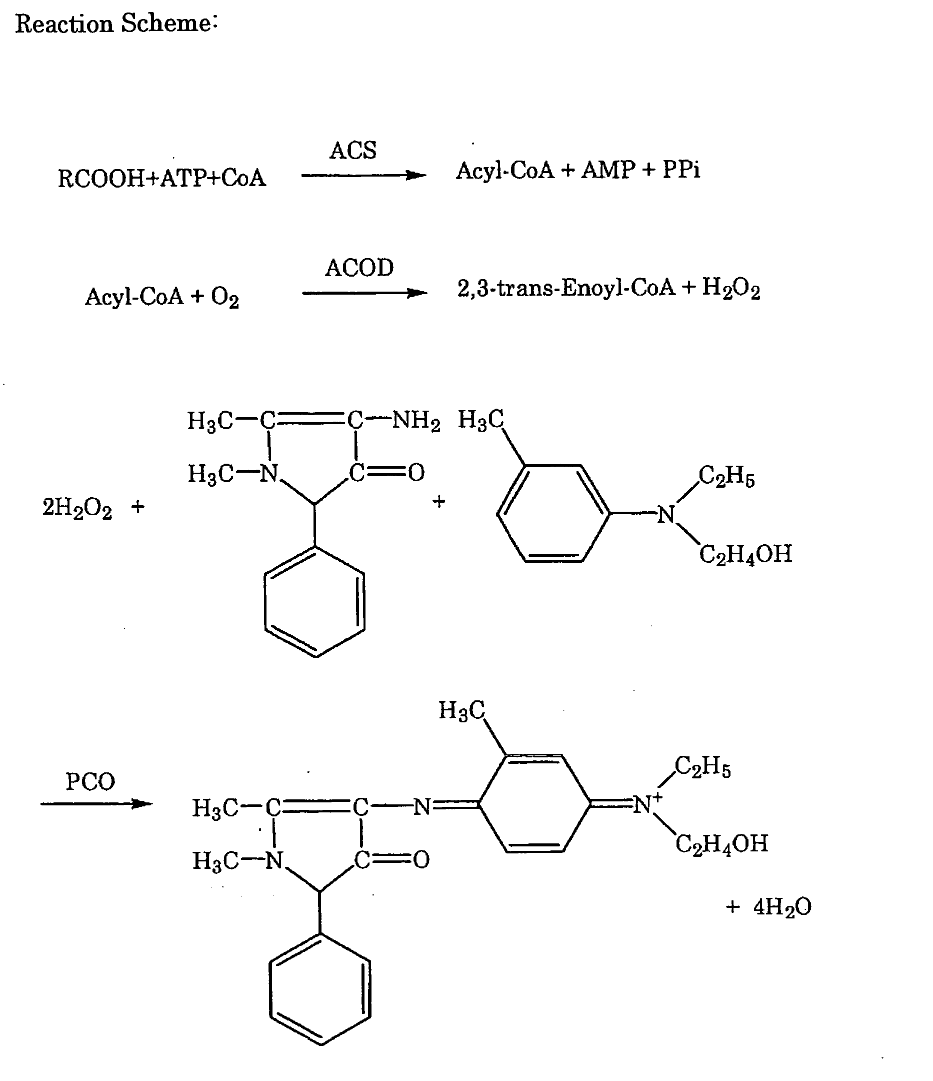

### Results:

### Variation of Blood Free Fatty Acid Value During Swimming Under Load

If the amino acid engine is effectively operated, it is expected that the combustion of the body fat is activated or promoted and this in turn leads to an increase in the blood free fatty acid value.

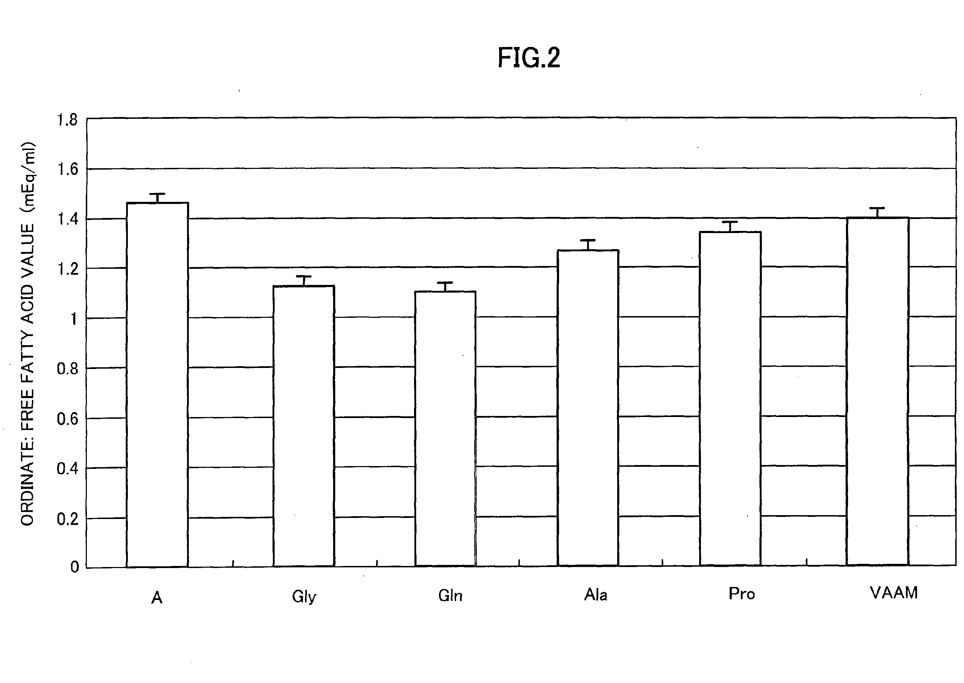

As has been shown in Fig. 2, the liquid nutrient A shows a blood free fatty acid value higher than that observed for VAAM and the blood free fatty acid value of the former is likewise higher than all of these values observed for other individual amino acids.

These results clearly indicate or suggest that the foregoing amino acid engine is effectively operated and that the metabolism of the body fat is accelerated.

### Industrial Applicability

The amino acid composition of the present invention permits the initiation of the combustion of the body fat within a short period of time and the promotion of the combustion thereof from the viewpoint of the combustion time and the amount of the consumed body fat and therefore, the composition is thus effective for the reduction of the fatigues accompanied by the physical exercise, the improvement of the performance of each individual and the dietary cure.

## Claims

1. An amino acid composition consisting essentially of (1) glycine; (2) at least one member selected from the group consisting of proline and alanine; and (3) at least one member selected from the group consisting of glutamine and glutamic acid.

2. An amino acid composition consisting essentially of glycine, proline, alanine, glutamine, and glutamic acid.

3. An amino acid composition consisting essentially of glycine, alanine, glutamine, and glutamic acid.

4. An amino acid composition consisting essentially of glycine, proline, glutamine, and glutamic acid.

5. An amino acid composition consisting essentially of glycine, proline, alanine, and glutamic acid.

6. An amino acid composition consisting essentially of glycine, proline, alanine, and glutamine.

7. An amino acid composition consisting essentially of glycine, alanine, and glutamine.

8. An amino acid composition consisting essentially of glycine, alanine, and glutamic acid.

9. An amino acid composition consisting essentially of glycine, proline, and glutamine.

10. An amino acid composition consisting essentially of glycine, proline, and glutamic acid.

11. The amino acid composition as set forth in any one of claims 1 to 10, wherein it further comprises CoA (coenzyme A).

12. The amino acid composition as set forth in any one of claims 1 to 11, wherein it further comprises acetyl CoA (acetyl-coenzyme A).

13. The amino acid composition as set forth in claims 1 to 12, wherein it further comprises pyruvic acid.
